# EUROPÄISCHE PATENTSCHRIFT

(11) **EP 3 234 101 B1**
(45) Veröffentlichungstag und Bekanntmachung des Hinweises auf die Patenterteilung: **20.02.2019**
(21) Anmeldenummer: 15816099.4
(22) Anmeldetag: 04.12.2015
(51) Int. Cl.: C12M 1/24, B01F 5/10, A61M 39/10, C12M 1/00, F16L 27/093, F16L 41/03

(54) **FLUID-VERSORGUNGSSCHNITTSTELLE, INSBESONDERE ZUR VERSORGUNG VON ZELLKULTURBEHÄLTERN, MIT WELCHSELND ZUR FLUIDLEITUNG ANSCHLIESSBAREN FLUIDKANÄLEN**
FLUID SUPPLY INTERFACE, IN PARTICULAR FOR SUPPLYING CELL CULTURE CONTAINERS, COMPRISING FLUID CHANNELS VARIABLY CONNECTABLE TO THE FLUID LINE
INTERFACE POUR L'ALIMENTATION EN FLUIDE, EN PARTICULIER POUR L'ALIMENTATION DE RÉCIPIENTS DE CULTURE CELLULAIRE, COMPRENANT DES CANAUX DE FLUIDE POUVANT SE FERMER ALTERNATIVEMENT POUR LA CONDUITE DU FLUIDE

(30) Priorität: 19.12.2014 DE 102014226692
(43) Veröffentlichungstag der Anmeldung: 25.10.2017
(73) Patentinhaber: Hamilton Bonaduz AG, 7402 Bonaduz (CH)
(72) Erfinder: GROSCH, Jens, 4051 Basel (CH); KISSLING, Tom, 4125 Riehen (CH); ZUMSTEIN, Thomas, 79576 Weil am Rhein (DE)
(74) Vertreter: Ruttensperger Lachnit Trossin Gomoll
(86) Internationale Anmeldenummer: PCT/EP2015/078717
(87) Internationale Veröffentlichungsnummer: WO 2016/096489

(56) Entgegenhaltungen:
- EP-A1- 1 672 265
- WO-A1-2011/090781
- WO-A1-2012/088014
- WO-A1-2014/114610
- DE-A1- 4 207 346
- DE-B3-102004 045 785
- GB-A- 2 433 304
- US-A1- 2009 111 180
- US-A1- 2011 223 076
- US-A1- 2014 076 454

## Beschreibung

Die vorliegende Erfindung betrifft eine Fluid-Versorgungsschnittstelle, insbesondere für eine Zellkulturanlage zur Einleitung eines Fluids in einen Zellkulturbehälter oder/und zur Ausleitung eines Fluids aus diesem, umfassend ein Leitungsbauteil mit einer ersten Ankopplungsformation zur vorübergehenden Ankopplung eines ersten Fluidkanals, vorzugsweise eines Fluidkanals eines Zellkulturbehälters, mit einer zweiten Ankopplungsformation zur vorübergehenden Ankopplung eines zweiten Fluidkanals, vorzugsweise eines Fluidkanals eines Fluidvorrats, und mit einer dritten Ankopplungsformation zur vorübergehenden oder dauerhaften Ankopplung eines dritten Fluidkanals, vorzugsweise eines Entsorgungskanals, welche Ankopplungsformationen jeweils von einem Fluidleitungsabschnitt durchsetzt sind, wobei in dem Leitungsbauteil eine Fluidleitungsanordnung ausgebildet ist, durch welche jeder Fluidleitungsabschnitt der ersten, der zweiten und der dritten Ankopplungsformation mit jedem Fluidleitungsabschnitt der anderen beiden Ankopplungsformationen zum Fluidtransport verbunden oder verbindbar ist.

Eine derartige Fluid-Versorgungsschnittstelle ist aus der WO 2014/114610 A bekannt. Diese Druckschrift zeigt insbesondere in ihren Figuren 2 und 3 eine gattungsgemäße Fluid-Versorgungsschnittstelle.

Wenngleich mit der bekannten Fluid-Versorgungsschnittstelle, mit welcher eine Vielzahl von Zellkulturbehältern oder auch andere fluidleitungsmäßig daran anschließbare Objekte in ihrem Fluidhaushalt manipulierbar sind, hervorragende Ergebnisse erzielt werden, besteht dennoch Raum für Verbesserung auch an dieser bekannten Fluid-Versorgungsschnittstelle.

Der Vorteil der gattungsgemäßen Fluid-Versorgungsschnittstellen liegt darin, dass eine einzige Fluid-Versorgungsschnittstelle an eine Vielzahl von unterschiedlichen Behältern, beispielsweise an eine Mehrzahl von Zellkulturbehältern, anschließbar ist, sodass mit einer einzigen Fluid-Versorgungsschnittstelle Fluid in die angeschlossenen Behälter eingeleitet, Fluid aus diesen ausgeleitet und entsorgt werden kann, und gegebenenfalls auch Produktionsergebnisse über die Fluidversorgungsschnittstelle aus den anschließbaren Behältern abgeführt, mithin unterschiedliche Zellkulturbehälter mit ein und derselben Fluid-Versorgungsschnittstelle "geerntet" werden können. Aufgrund der an der Fluid-Versorgungsschnittstelle vorgesehenen Ankopplungsformationen zur Ankopplung von Fluidkanälen, an welche auch ein Reinigungsfluidvorrat ankoppelbar ist, ist eine ausreichende Reinigungsmöglichkeit der in der Fluid-Versorgungsschnittstelle vorgesehenen Fluidleitungsanordnung gegeben, sodass Kontaminationen, insbesondere hochgefährliche Kreuzkontaminationen, trotz der 1:n-Zuordnung der Fluid-Versorgungsschnittstelle zu einer Mehrzahl von Zellkulturbehältern nicht zu befürchten sind.

Ein verbesserungswürdiger Aspekt der bekannten Fluid-Versorgungsschnittstelle ist deren Bauraumforderung. Die bekannte Fluid-Versorgungsschnittstelle benötigt mit zunehmender Anzahl von Ankopplungsformationen bzw. daran angekoppelten Fluidkanälen zunehmend Bauraum.

Es ist daher Aufgabe der vorliegenden Erfindung, die eingangs genannte Fluid-Versorgungsschnittstelle hinsichtlich ihres Bauraumbedarfs zu verbessern, sodass sie bei gleicher Anzahl an angekoppelten oder ankoppelbaren Fluidkanälen weniger Bauraum in Anspruch nimmt als die bereits bekannte Fluid-Versorgungsschnittstelle.

Diese Aufgabe wird erfindungsgemäße gelöst durch eine gattungsgemäße Fluid-Versorgungsschnittstelle, wie sie eingangs beschrieben ist, bei welcher die Fluid-Versorgungsschnittstelle weiter einen relativ zum Leitungsteil beweglichen Fluidkanalträger aufweist, welcher wenigstens zwei gesondert voneinander ausgebildete und mit unterschiedlichen Fluidkanälen zum Fluidtransport verbundene, jeweils von einem Fluidkanalabschnitt durchsetzte Fluidkanalträger-Anschlussformationen aufweist, und wobei eine der drei Ankopplungsformationen als Wechsel-Ankopplungsformation zur vorübergehenden Herstellung einer gemeinsamen Fluidleitungsstrecke mit einer durch Relativbewegung von Leitungsbauteil und Fluidkanalträger auswählbaren Fluidkanalträger-Anschlussformation ausgebildet ist.

Im Gegensatz zur bekannten gattungsgemäßen Fluid-Versorgungsschnittstelle, bei welcher alle daran angekoppelten Fluidkanäle - mit Ausnahme der bestimmungsgemäß nur vorübergehend ankoppelbaren Zellkulturbehälter-Fluidkanäle - fest mit der Fluid-Versorgungsschnittstelle verbunden sind, ist bei der erfindungsgemäßen Fluid-Versorgungsschnittstelle vorgesehen, neben der Ankopplungsformation für den Zellkulturbehälter wenigstens eine weitere Ankopplungsformation als Wechsel-Ankopplungsformation auszugestalten, mit welcher ein Fluidkanal lediglich vorübergehend eine gemeinsame Fluidleitungsstrecke bildet.

Hierzu weist der Fluidkanalträger wenigstens zwei gesondert voneinander ausgebildete und mit unterschiedlichen Fluidkanälen zum Fluidtransport verbundene Fluidkanalträger-Anschlussformationen auf, von welchen jede zur Herstellung einer gemeinsamen Fluidleitungsstrecke mit der Wechsel-Ankopplungsformation des Leitungsbauteils vorübergehend koppelbar ist. Diejenige Fluidkanalträger-Anschlussformation, welche mit der Wechsel-Ankopplungsformation des Leitungsbauteils eine gemeinsame Fluidleitungsstrecke bilden soll, kann in einfacher Weise durch Relativbewegung von Fluidkanalträger und Leitungsteil ausgewählt werden. Somit können an der erfindungsgemäßen Fluid-Versorgungsschnittstelle in vorteilhafter Weise eine größere Anzahl an zu ihr hin oder/und von ihr weg führenden Fluidkanälen dauerhaft mit ihr verbunden sein, als Ankopplungsformationen am Leitungsbauteil vorgesehen sind. Somit können Ankopplungsformationen am Leitungsbauteil eingespart werden, was zu der gewünschten Bauraumeinsparung führt.

Über den Fluidkanalträger sind dabei Fluidkanäle zwar fest mit der erfindungsgemäßen Fluid-Versorgungsschnittstelle verbunden, jedoch nicht fest mit der Wechsel-Ankopplungsformation des Leitungsbauteils.

Die Auswahl einer Fluidkanalträger-Anschlussformation als Kopplungs-Anschlussformation, welche mit der Wechsel-Ankopplungsformation eine gemeinsame Fluidleitungsstrecke bilden soll, kann in einfacher Weise konstruktiv dadurch angelegt sein, dass der Fluidkanalträger wenigstens zwei Betriebsrelativstellungen aufweist, in denen jeweils eine andere Fluidkanalträger-Anschlussformation als Kopplungs-Anschlussformation ausgewählt und der Wechsel-Ankopplungsformation stärker angenähert ist als die jeweils andere Fluidkanalträger-Anschlussformation.

Dabei soll bewusst die Annäherung der ausgewählten Kopplungs-Anschlussformation auf Seiten des Fluidkanalträgers und der Wechsel-Ankopplungsformation auf Seiten des Leitungsbauteils aneinander allgemein verstanden werden. Der Zustand stärkerer Annäherung der Kopplungs-Anschlussformation an die Wechsel-Ankopplungsformation kann beispielsweise eine Kopplungsbereitschaftsstellung sein, in der sich dann die Kopplungs-Anschlussformation befindet. In dieser Kopplungsbereitschaftsstellung fluchten bevorzugt der die Kopplungs-Anschlussformation durchsetzende Fluidkanalabschnitt und der die Wechsel-Ankopplungsformation durchsetzende Fluidleitungsabschnitt miteinander, sodass ausgehend von dieser Kopplungsbereitschaftsstellung eine tatsächliche Fluidleitungsfähigkeit durch die bezeichneten Abschnitte: Fluidkanalabschnitt und Fluidleitungsabschnitt, einfach und schnell herstellbar sein kann.

Alternativ oder zusätzlich kann sich die Kopplungs-Anschlussformation in dem Zustand stärkerer Annäherung an die Wechsel-Ankopplungsformation in einer Kopplungsstellung befinden, in welcher der die Kopplungs-Anschlussformation durchsetzende Fluidkanalabschnitt und der die Wechsel-Ankopplungsformation durchsetzende Fluidleitungsabschnitt zur Bildung einer gemeinsamen die Kopplungs-Anschlussformation und die Wechsel-Ankopplungsformation durchsetzenden Fluidleitungsstrecke gekoppelt sind. In diesem Falle ist die gemeinsame Fluidleitungsstrecke, an der die beteiligten Abschnitte: Fluidkanalabschnitt und Fluidleitungsabschnitt, der Kopplungs-Anschlussformation und der Wechsel-Ankopplungsformation beteiligt sind, bereits hergestellt.

Wenngleich grundsätzlich daran gedacht sein kann, die Wechsel-Ankopplungsformation beweglich am Leitungsbauteil vorzusehen, um mit der so geschaffenen Beweglichkeit eine gemeinsame Fluidleitungsstrecke zwischen Kopplungs-Anschlussformation und der Wechsel-Ankopplungsformation herzustellen, ist es bevorzugt, die Beweglichkeit an der Kopplungs-Anschlussformation vorzusehen. Folgerichtig ist es bevorzugt, dass die jeweilige Kopplungs-Anschlussformation zwischen der Kopplungsbereitschaftsstellung und der Kopplungsstellung verlagerbar ist. Diese Verlagerbarkeit ist bevorzugt eine Verlagerbarkeit relativ zum Fluidkanalträger, an welchem die Kopplungs-Anschlussformation als Fluidkanalträger-Anschlussformation aufgenommen ist und auch relativ zum Leitungsbauteil, an welchem sich die Wechsel-Ankopplungsformation befindet. Dann, wenn der die Kopplungs-Anschlussformation durchsetzende Fluidkanalabschnitt und der die Wechsel-Ankopplungsformation durchsetzende Fluidleitungsabschnitt in der Kopplungsbereitschaftsstellung miteinander fluchten, kann in bevorzugter Weise die jeweils ausgewählte Kopplungs-Anschlussformation ohne Änderung der Relativstellung von Fluidkanalträger und Leitungsbauteil zur Kopplungsstellung hin und wieder zurück verlagerbar sein.

Bevorzugt erfolgt die Verlagerung des Kopplungs-Anschlussformation zwischen der Kopplungsbereitschaftsstellung und der Kopplungsstellung relativ zum Fluidkanalträger längs einer Kopplungsbahn, die aus Gründen möglichst einfacher Verlagerung bevorzugt wenigstens abschnittsweise, besonders bevorzugt vollständig, eine geradlinige Kopplungsachse sein kann.

Weiter sind die Fluidkanalträger-Anschlussformationen und unter diesen die zur Kopplung mit der Wechsel-Ankopplungsformation ausgewählte Kopplungs-Anschlussformation gemeinsam mit dem Fluidkanalträger relativ zum Leitungsbauteil längs einer Auswahlbahn beweglich. Die Auswahlbahn ist durch ein Führungsmittel definiert, das die Relativbewegung von Fluidkanalträger und Leitungsbauteil zur Auswahl einer Fluidkanalträger-Anschlussformation als die Kopplungs-Anschlussformation führt.

Um eine Verwechslung der beiden Bewegungsbahnen auszuschließen, unterscheiden sich Kopplungsbahn und Auswahlbahn voneinander, sodass eine die jeweiligen Bewegungen veranlassende Bedienperson klar zwischen der Auswahl einer Fluidkanalträger-Anschlussformation als der Kopplungs-Anschlussformation und der Herstellung einer gemeinsamen Fluidleitungsstrecke der Kopplungs-Anschlussformation mit der Wechsel-Ankopplungsformation unterscheiden kann.

Bevorzugt sind die Kopplungsbahn und die Auswahlbahn orthogonal zueinander orientiert, sodass möglichst die Bewegung längs einer der Bahnen keine Bewegungskomponente längs der jeweils anderen Bahn aufweist. Orthogonale Bewegungen im Sinne der vorliegenden Anmeldung sind dabei nicht nur translatorische Bewegungen mit zueinander orthogonalen translatorischen Bewegungsrichtungen, sondern auch rotatorische Bewegungen um zueinander orthogonale Rotationsachsen sowie eine translatorische Bewegung längs einer Bewegungsachse und eine rotatorische Bewegung um eine zur Bewegungsachse parallele oder mit dieser kollinearen Rotationsachse. Diese Aufzählung ist ausdrücklich nicht vollständig, sodass noch weitere Konstellationen von translatorischer und rotatorischer Bewegung zu zueinander orthogonalen Bewegungsbahnen führen können.

Um eine mit der erfindungsgemäßen Fluid-Versorgungsschnittstelle arbeitenden Bedienperson möglichst zu entlasten, kann die Fluid-Versorgungsschnittstelle ein Kraftgerät aufweisen, durch welches die Kopplungs-Anschlussformation von der Kopplungsbereitschaftsstellung in die Kopplungsstellung oder/und umgekehrt verlagerbar ist. Mit diesem Kraftgerät wird eine Bedienperson von manueller Handhabung entlastet. Überdies kann durch das Kraftgerät eine wiederholbare Verlagerungskraft bereitgestellt werden, welche einen Kopplungserfolg zwischen der Kopplungs-Anschlussformation und der Wechsel-Ankopplungsformation mit ausreichender Sicherheit gewährleistet. Als Kraftgeräte kommen elektromotorische Antriebe, Elektromagnete, pneumatische oder auch hydraulische Antriebe in Frage. Bevorzugt wird das Kraftgerät ein elektromagnetischer Antrieb sein, gegebenenfalls unter Zwischenanordnung eines Getriebes.

Das Kraftgerät kann dazu ausgebildet sein, während der Dauer der Herstellung der gemeinsamen Fluidleitungsstrecke eine ausreichende Kopplungskraft bereitzustellen, um die Kopplungs-Anschlussformation und die Wechsel-Ankopplungsformation fluiddicht miteinander zu koppeln. Aus Gründen einer bevorzugten Energieeinsparung kann gemäß einer vorteilhaften Weiterbildung der vorliegenden Erfindung jedoch vorgesehen sein, dass die Kopplungs-Anschlussformation in der Kopplungsstellung relativ zum Leitungsbauteil und damit in der Regel auch relativ zum Fluidkanalträger verriegelbar ist. Diese vorteilhafte Weiterbildung der Verriegelbarkeit kann unabhängig von der oben beschriebenen Bereitstellung des Kraftgeräts erfolgen.

Zur Bereitstellung einer definierten Ausgangsposition sowie zur Vereinfachung des Aufbaus des Kraftgeräts kann die Kopplungs-Anschlussformation bzw. jede Fluidkanalträger-Anschlussformation gegen die Vorspannkraft einer Vorspanneinrichtung zwischen der Kopplungsbereitschaftsstellung und der Kopplungsstellung in eine Richtung verlagerbar sein. In diesem Falle braucht das Kraftgerät nur in einer Richtung Kraft aufwenden, während in die entgegengesetzte Richtung eine Rückstellung durch die Vorspanneinrichtung erfolgen kann. Die Vorspanneinrichtung kann eine Feder sein.

Die Fluidleitungsanordnung kann die Fluidleitungsabschnitte der ersten, der zweiten und der dritten Ankopplungsformation des Leitungsbauteils parallel miteinander verbinden, d. h. zwei Ankopplungsformationen sind miteinander durch die Fluidleitungsanordnung derart verbunden, dass übrige Ankopplungsformationen durch die paarweise Verbindung zweier Ankopplungsformationen im Wesentlichen umgangen werden. Eine bevorzugte parallele Verbindung der ersten, der zweiten und der dritten Ankopplungsformation ist eine sternförmige Ausbildung der Fluidleitungsanordnung.

Alternativ kann die Fluidleitungsanordnung die Fluidleitungsabschnitte der ersten, der zweiten und der dritten Ankopplungsformation auch seriell miteinander verbinden. Dies bedeutet, dass unter Umständen eine Ziel-Anschlussformation von einer von einer Start-Anschlussformation ausgehenden Fluidströmung erst erreicht wird, nachdem die Fluidströmung eine zwischen Start- und Ziel-Anschlussformation gelegene weitere Anschlussformation passiert hat. Die serielle Verbindung ist bevorzugt bogenförmig oder polygonal und besonders bevorzugt ringförmig, also geschlossenringförmig, im Leitungsbauteil ausgebildet. Mit "ringförmig" ist dabei jedoch nicht notwendigerweise ein bogenförmiger oder gar kreisringförmiger Verlauf bezeichnet. Ein ringförmiger Verlauf kann auch durch Aneinanderreihung geradliniger Leitungsabschnitte realisiert sein, sofern diese zu einer geschlossen umlaufenden Leitung zusammengeführt sind.

Damit ein Ventilkörper, welcher über weite Betriebszeitabschnitte einen eine Ankopplungsformation durchsetzenden Fluidleitungsabschnitt für eine Fluiddurchleitung sperrt, dann die Fluidleitung nicht stört, wenn die Fluidleitung durch den die Ankopplungsformation des Ventilkörpers durchsetzenden Fluidleitungsabschnitt hindurchgeführt werden soll, ist es bevorzugt, wenn die Fluidleitungsanordnung im Bereich wenigstens einer Ankopplungsformation eine Aufnahmeausnehmung aufweist, welche zur vorübergehenden Aufnahme des Ventilkörpers ausgebildet ist. Bevorzugt kann diese Aufnahmeausnehmung raumsparend in einer Richtung orthogonal zur Öffnungsfläche einer Mündung des die Ankopplungsformation durchsetzenden Fluidleitungsabschnitts in der wenigstens einen Ankopplungsformation in das Leitungsbauteil hinein versetzt vorgesehen sein. Dies ist vor allem bei der seriellen Verbindung der Ankopplungsformationen konstruktiv leicht realisierbar.

Bei der parallelen, also bevorzugt sternförmigen Ausbildung der Fluidleitungsanordnung kann die Aufnahmeausnehmung seitlich am Fluidleitungsabschnitt vorgesehen sein, sodass der Ventilkörper orthogonal oder wenigstens mit einer Bewegungskomponente orthogonal zu dem die jeweilige Ankopplungsformation durchsetzenden Fluidleitungsabschnitt in die Aufnahmeausnehmung hinein und aus dieser heraus zurückbewegt werden kann.

Um den Ventilkörper, welcher auch in der Aufnahmeausnehmung den Kräften einer an ihm vorbeiströmenden Fluidströmung ausgesetzt sein kann, möglichst sicher in der Aufnahmeausnehmung halten zu können, ist es gemäß einer vorteilhaften Weiterbildung der Erfindung bevorzugt, dass die Aufnahmeausnehmung wenigstens abschnittsweise komplementär zu einem Oberflächenabschnitt des Ventilkörpers ausgebildet ist.

Um jegliche Überlegungen hinsichtlich der Orientierung des Ventilkörpers überflüssig zu machen, ist eine Ventilkugel als Ventilkörper bevorzugt, sodass dann die Aufnahmeausnehmung bevorzugt konkav-teilsphärisch ausgebildet sein kann.

Zur möglichst fluiddichten Kopplung einer Ankopplungsformation mit einer Anschlussformation kann gemäß einer bevorzugten Weiterbildung vorgesehen sein, dass die Mündung des Fluidleitungsabschnitts an der Wechsel-Ankopplungsformation, bevorzugt auch an wenigstens einer weiteren Ankopplungsformation, von einer Dichtung oder von einer Anlagefläche, vorzugsweise von einer ebenen Anlagefläche, welche zur Anlage einer an einer Anschlussformation vorgesehenen Dichtung an ihr ausgebildet ist, umgeben ist.

Weiter kann insbesondere in dem oben dargelegten bevorzugten Anwendungsbeispiel der vorliegend beschriebenen Fluid-Versorgungsschnittstelle in einer Zellkulturanlage, also ausgebildet für ein Zusammenwirken mit einem Zellkulturbehälter, eine feste Verbindung von Ankopplungsformation und Anschlussformation wünschenswert sein. Deswegen kann wenigstens eine der Ankopplungsformationen dauerhaft mit einer Anschlussformation zur Bildung einer gemeinsamen die Anschlussformation und die Ankopplungsformation durchsetzenden Fluidleitungsstrecke gekoppelt sein. Dies kann beispielsweise für eine in einen Abfall-Behälter (waste) führenden Fluidkanal von Vorteil sein, da die Verbindung zum Abfallbehälter, oder allgemein zu einer Fluidsenke, stets benötigt sein kann.

Ein weiterer Vorteil in der festen Anordnung wenigstens eines Fluidkanals bzw. dessen Anschlussformation an eine Ankopplungsformation des Leitungsbauteils kann darin liegen, dass mit der dauerhaften Kopplung ein Fluidleitungsabschnitt bereitgestellt werden kann, welcher den Fluidleitungsabschnitt an der Wechsel-Ankopplungsformation umgeht. Auch dies ist beispielsweise für zu entsorgendes Abfall-Fluid von Vorteil, sodass dieses nicht durch den Fluidleitungsabschnitt der Wechsel-Ankopplungsformation geleitet werden muss, durch den später möglicherweise steriles Nährfluid zurück in den Zellkulturbehälter geleitet wird. Es sei dabei ausdrücklich klargestellt, dass die vorliegende Fluid-Versorgungsschnittstelle sich in Versuchen als so gut durch Spülungen mit Reinigungsfluid zu reinigen herausgestellt hat, dass selbst die Verwendung gemeinsamer Fluidleitungsabschnitte zur Durchleitung von Abfall-Fluid und Nährfluid problemlos möglich wäre. Die Trennung dieser Fluidleitungswege erhöht also lediglich die ohnehin schon ausreichende Betriebssicherheit der Fluid-Versorgungsschnittstelle.

Es wurde oben bereits Bezug auf einen Ventilkörper genommen. Die unterschiedlichen am Leitungsbauteil vorgesehenen Ankopplungsformationen und die diese verbindende Fluidleitungsanordnung sollte zur Erhöhung der mit der vorliegenden Fluid-Versorgungsschnittstelle erzielbaren Betriebshygiene für unterschiedliche Fluide unterschiedliche Fluidleitungsabschnitte bereitstellen können. Dies kann in vorteilhafter Weise dadurch geschehen, dass eine mit einer Ankopplungsformation zur Bildung einer gemeinsamen Fluidleitungsstrecke koppelbare oder gekoppelte Anschlussformation einen Ventilsitz und einen Ventilkörper aufweist, welcher in einem Betriebszustand mit für eine Fluiddurchleitung gesperrtem Fluidkanalabschnitt der Anschlussformation auf dem Ventilsitz ruht.

Vorteilhafterweise kann der Ventilsitz einen Magneten aufweisen, mit welchem der vorzugsweise wenigstens teilweise oder sogar vollständig aus ferromagnetischem Material gebildete Ventilkörper in eine Sperrstellung vorspannbar ist, in welcher der Ventilkörper auf dem Ventilsitz ruht. Für eine möglichst homogene Kraftverteilung bei der Vorspannung des Ventilkörpers zum Ventilsitz hin kann es vorteilhaft sein, einen ringförmigen Magneten zu verwenden, wobei weiter bevorzugt die den Ring orthogonal zu einer Ringebene, in der sich der Ring erstreckt, durchsetzende Ringachse vorteilhafterweise mit der Verlaufsrichtung des die jeweilige Anschlussformation durchsetzenden Fluidkanalabschnitts zusammenfällt, also bevorzugt kollinear ist.

Bevorzugt ist der den Ventilkörper in die Schließstellung vorspannende Magnet von Fluid durchströmbar, sodass er mit geringem Bauraumaufwand in oder nahe der den Ventilsitz aufweisenden Anschlussformation vorgesehen sein kann.

Da jedoch nicht dauerhaft alle möglichen Fluidleitungsabschnitte abgesperrt zu sein brauchen, kann daran gedacht sein, dass ein oder mehrere Ventilkörper gemeinsam von mehreren Ankopplungsformationen benutzt werden. Dies kann beispielsweise konstruktiv dadurch ermöglicht werden, dass wenigstens ein von der Ankopplungsformation ausgehender und in Richtung zu einer weiteren Ankopplungsformation hin verlaufender Verlagerungsabschnitt der Fluidleitungsanordnung derart bemessen ist, dass er eine Verlagerung des Ventilkörpers von der Ankopplungsformation weg, vorzugsweise bis zu einer weiteren Ankopplungsformation, und zu dieser zurück gestattet.

Eine besonders vorteilhafte Möglichkeit, die Ventilkörper berührungslos und damit hochhygienisch zwischen zwei Stellungen zu schalten, von welchen der Ventilkörper in einer einen Fluiddurchgang durch den Ventilsitz sperrt und in einer anderen den Fluiddurchgang durch den Ventilsitz gestattet, kann dadurch realisiert sein, dass der Ventilkörper ferromagnetisches Material aufweist, insbesondere aus ferromagnetischem Material hergestellt ist, und dass die Fluid-Versorgungsschnittstelle eine magnetische Schalteinrichtung mit lokal an den Ankopplungsformationen veränderbarer magnetischer Feldstärke aufweist, mit welcher der Ventilkörper von dem Ventilsitz lösbar ist.

Oben wurden bereits die unterschiedlichen Relativbewegungsbahnen, nämlich die Auswahlbahn und die Kopplungsbahn, beschrieben. Im Hinblick auf die zu lösende Aufgabe, nämlich die Bereitstellung der vorliegenden Fluid-Versorgungsschnittstelle mit verglichen mit dem Stand der Technik reduziertem Bauraum, ist es bevorzugt, wenn der Fluidkanalträger relativ zum Leitungsbauteil um eine Rotationsachse, vorzugsweise um eine zur Verlaufsrichtung des die Wechsel-Ankopplungsformation durchsetzenden Fluidleitungsabschnitts parallele Rotationsachse drehbar ist, oder relativ zum Leitungsbauteil längs einer Translationsachse, vorzugsweise längs einer zur Verlaufsrichtung des die Wechsel-Ankopplungsformation durchsetzenden Fluidleitungsabschnitts orthogonale Translationsachse verschiebbar ist. Da die vorliegend diskutierte Fluid-Versorgungsschnittstelle vor allem in Zellkulturanlagen mit wenigstens einem Zellkulturbehälter vorteilhaft anwendbar ist, betrifft die vorliegende Erfindung auch eine Zellkulturanlage mit wenigstens einem Zellkulturbehälter, mit wenigstens zwei gesondert vorgesehenen Fluidvorräten, mit einer Fluidentsorgungssenke und mit einer Fluid-Versorgungsschnittstelle, wie sie oben beschrieben und gegebenenfalls weitergebildet ist, wobei der Zellkulturbehälter eine Anschlussformation aufweist, wobei die Ankopplungsverbindungen des Fluidkanalträgers fluidleitend mit den Fluidvorräten verbunden sind, wobei die erste Ankopplungsformation des Leitungsbauteils zur Herstellung einer vorübergehenden Fluidtransportverbindung mit der Zellkulturbehälter-Anschlussformation ausgebildet ist, die zweite Ankopplungsformation des Leitungsbauteils als die Wechsel-Ankopplungsformation zur Herstellung einer vorübergehenden Fluidtransportverbindung mit einer der Fluidkanalträger-Anschlussformationen ausgebildet ist und die dritte Ankopplungsformation des Leitungsbauteils zur Herstellung einer vorübergehenden oder dauerhaften Fluidtransportverbindung mit der Fluidentsorgungssenke ausgebildet ist.

Bevorzugt umfasst die Zellkulturanlage eine Mehrzahl von Zellkulturbehältern und eine verglichen mit der Anzahl an Zellkulturbehältern geringere Anzahl an Fluid-Versorgungsschnittstellen, bevorzugt nur eine Fluid-Versorgungsschnittstelle.

Der Fluidkanalträger kann mehr als zwei Fluidkanalträger-Anschlussformationen aufweisen, beispielsweise drei, vier, fünf, sechs oder sogar mehr als sechs Fluidkanalträger-Anschlussformationen, wobei gilt: Bei einer Anzahl von mehr als drei Fluidkanalträger-Anschlussformationen benötigt eine rotatorische Relativbeweglichkeit des Fluidkanalträgers relativ zum Leitungsbauteil verglichen einer mit einer translatorischen Relativbeweglichkeit den geringeren Bau- und Bewegungsraum. Die Fluidkanalträger-Anschlussformationen mit den sie durchsetzenden Fluidkanalabschnitten sind vorzugsweise parallel zur Relativrotationsachse des Fluidkanalträgers relativ zum Leitungsbauteil mit radial gleichem Abstand zur Relativrotationsachse vorgesehen.

Der Fluidkanalträger kann selbst wiederum motorisch angetrieben zur Relativbewegung längs der Auswahlbahn sein. Hierzu kann die Fluid-Versorgungsschnittstelle einen Bewegungsantrieb aufweisen, welcher Bewegung und Kraft übertragend mit dem Fluidkanalträger gekoppelt am Leitungsbauteil oder an einem fest mit dem Leitungsbauteil gekoppelten Bauteil, wie etwa Gestell und dgl., vorgesehen sein kann.

Die vorliegende Erfindung wird nachfolgend anhand der beiliegenden Zeichnungen näher erläutert. Es stellt dar:
- **Figur** 1: eine erste Ausführungsform einer erfindungsgemäßen Fluid-Versorgungsschnittstelle mit angekoppeltem Zellkulturbehälter in perspektivischer Ansicht,
- **Figur 2**: eine Längsschnittansicht durch die Ausführungsform von Figur 1,
- **Figur 3**: die erste Ausführungsform von Figur 1 mit abgekoppeltem Zellkulturbehälter,
- **Figur 4**: eine zweite Ausführungsform einer erfindungsgemäßen Fluid-Versorgungsschnittstelle mit angekoppeltem Zellkulturbehälter,
- **Figur 5**: eine Längsschnittansicht durch die zweite Ausführungsform von Figur 4,
- **Figur 6**: das Leitungsbauteil der Fluid-Versorgungsschnittstelle von Figur 5 in einer Detaildarstellung und
- **Figur 7**: ein alternatives Leitungsbauteil für die zweite Ausführungsform.

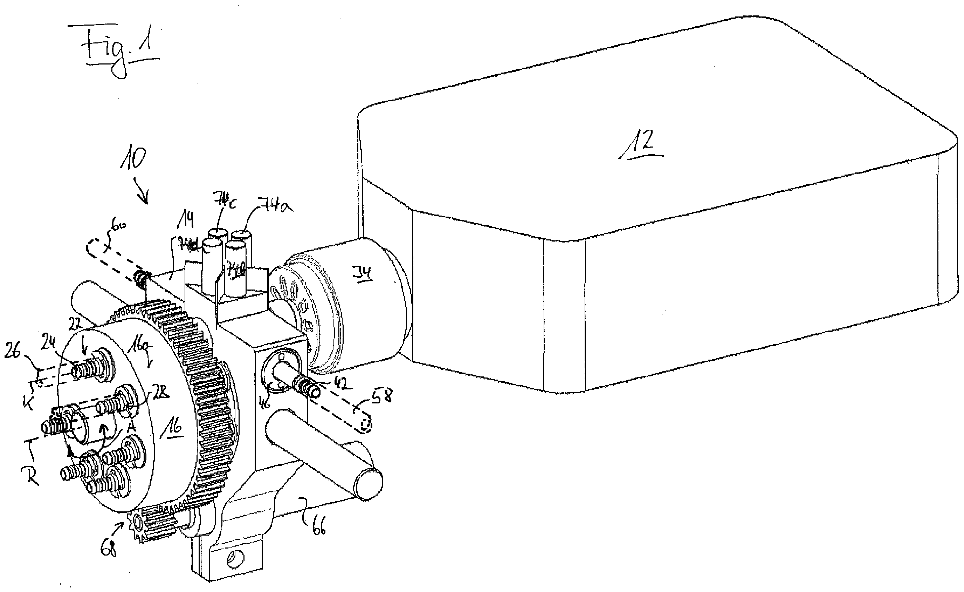

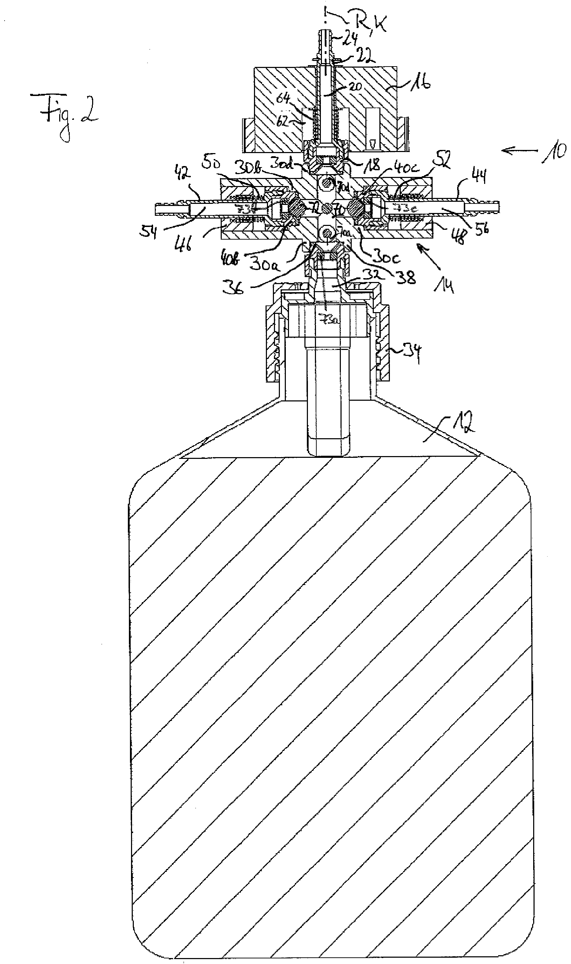

In Figur 1 ist eine erste Ausführungsform einer Fluid-Versorgungsschnittstelle der vorliegenden Erfindung allgemein mit 10 bezeichnet. An diese Fluid-Versorgungsschnittstelle 10 ist in den Figuren 1 und 2 ein Zellkulturbehälter 12 angekoppelt.

Die Fluid-Versorgungsschnittstelle 10 umfasst ein Leitungsbauteil 14, an welchem ein Fluidkanalträger 16 um eine Rotationsachse R drehbar vorgesehen ist.

An dem Fluidkanalträger 16 ist eine Mehrzahl - im vorliegenden Beispiel genau sechs - von Fluidkanalträger-Anschlussformationen 18 (siehe Figur 2) aufgenommen, welche jeweils von einem Fluidkanalabschnitt 20 durchsetzt sind.

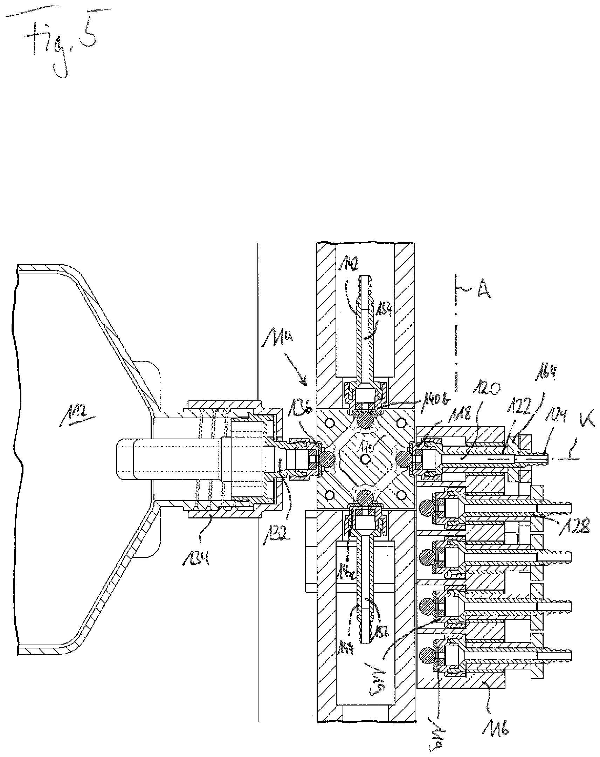

Die Fluidkanalträger-Anschlussformation 18, wie die übrigen Fluidkanalträger-Anschlussformationen 19 (siehe in Figur 5 als "119") auch, ist an einem Fluidkanalträger-Anschlussstutzen 22 ausgebildet, welcher an seinem von der Fluidkanalträger-Anschlussformation 18 fernliegenden Längsende 24 zum Anschluss eines Fluidleitungsmittels 26, beispielsweise in Form eines flexiblen Schlauchs, ausgebildet ist.

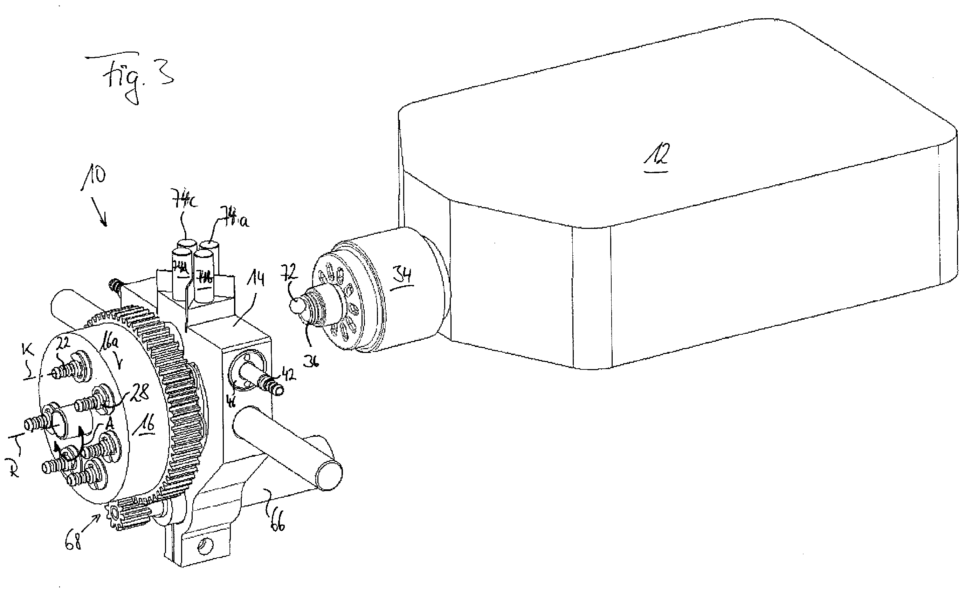

In den Figuren 1 und 3 ist zur Unterscheidung der Fluidkanalträger-Anschlussstutzen 22 voneinander ein weiterer Anschlussstutzen mit 28 bezeichnet. Die Fluidkanalträger-Anschlussstutzen sind bevorzugt im Wesentlichen identisch aufgebaut.

Wie man in Figur 2 erkennt, weist das Leitungsbauteil 14 eine Mehrzahl von - im vorliegenden Beispiel vier - Ankopplungsformationen 30a bis 30d auf. Die Ankopplungsformation 30a ist dabei zur vorübergehenden Ankopplung eines Fluidkanals 32 ausgebildet, der in einem Deckel 34 des Zellkulturbehälters 12 in an sich bekannter Weise vorgesehen ist. Der Deckel 34 weist zur Ankopplung an die Ankopplungsformation 30a eine Zellkulturbehälter-Anschlussformation 36 auf, welche im Wesentlichen vorteilhaft in Übereinstimmung mit den übrigen Anschlussformationen, beispielsweise der Fluidkanalträger-Anschlussformation 18, ausgebildet ist.

Die Ankopplungsformationen 30a bis 30d weisen jeweils bevorzugt einen umlaufenden Bund auf, in welchen die zugeordnete Anschlussformation zur Ankopplung an die jeweilige Ankopplungsformation 30a bis 30d eingetaucht ist. Der Übersichtlichkeit halber ist lediglich der Bund 38 der Ankopplungsformation 30a mit einem Bezugszeichen versehen.

Die Ankopplungsformationen 30b und 30c sind im vorliegenden Beispiel fest und dauerhaft mit Anschlussformationen 40b und 40c zur Bildung einer gemeinsamen Fluidleitungsstrecke gekoppelt. Hierzu kann das Leitungsbauteil 14 einen von Fluidkanalstutzen 42 und 44 durchsetzten Stützkörper 46 bzw. 48 aufweisen, an welchem sich eine Spannfeder 50 bzw. 52 abstützt, die die Anschlussformationen 40b bzw. 40c zu den jeweiligen Ankopplungsformationen 30b bzw. 30c hin vorspannt. Die Stützkörper 46 und 48 sind hierzu als Federwiderlager bevorzugt fest mit dem Leitungsbauteil 14 verbunden.

Die Anschlussformationen 40b und 40c sind von einem Fluidkanalabschnitt 54 bzw. 56 durchsetzt, welcher von den Anschlussstutzen 42 und 44 definiert sind, die an ihren von der Anschlussformation 40b bzw. 40c jeweils fernliegenden Längsenden wiederum zur Anbringung je eines Fluidleitungsmittels - hier eines Schlauchs 58 bzw. 60 - ausgebildet sind.

Im Gegensatz zu den fest mit den Ankopplungsformationen 30b und 30c gekoppelten Anschlussformationen 40b und 40c kann die Ankopplungsformation 30d mit unterschiedlichen Anschlussformationen des Fluidkanalträgers 16 zur Herstellung einer gemeinsamen Fluidleitungsstrecke gekoppelt werden. Die Ankopplungsformation 30d wird daher nachfolgend als Wechsel-Ankopplungsformation 30d bezeichnet.

Da in den Figuren 1 bis 3 die nur in Figur 2 erkennbare Fluidkanalträger-Anschlussformation 18 des Fluidkanalträgers 16 der Wechsel-Ankopplungsformation 30d am stärksten angenähert ist, mithin diese als Kopplungs-Anschlussformation 18 zur Kopplung mit der Wechsel-Ankopplungsformation 30d ausgewählt ist, ist nachfolgend die Fluidkanalträger-Anschlussformation 18 als Kopplungs-Anschlussformation 18 bezeichnet. Diese befindet sich in Figur 2 in einer Kopplungsstellung, in welcher sie mit der Wechsel-Ankopplungsformation 30d zur Bildung einer gemeinsamen Fluidleitungsstrecke, die dann die Kopplungs-Anschlussformation 18 und die Wechsel-Ankopplungsformation 30d durchsetzt, gekoppelt ist.

Die Kopplungs-Anschlussformation 18 von Figur 2 ist längs einer zur Rotationsachse R vorzugsweise parallelen Kopplungsbahn K, insbesondere Kopplungsachse K, zwischen der in Figur 2 gezeigten Kopplungsstellung und einer Kopplungsbereitschaftsstellung verstellbar, in welcher die Kopplungs-Anschlussformation 18 längs der Kopplungsbahn K mit Abstand von der Wechsel-Ankopplungsformation 30d angeordnet ist. Die Kopplungs-Anschlussformation 18 befindet sich dann tiefer in dem koaxialen Aufnahmeraum 62 im Fluidkanalträger aufgenommen, sodass der Fluidkanalträger 16 um die Rotationsachse R rotiert werden kann, ohne dass eine Kollision zwischen der Kopplungs-Anschlussformation 18 oder einer anderen Fluidkanalträger-Anschlussformation mit der Wechsel-Ankopplungsformation 30d zu befürchten ist. In der Kopplungsbereitschaftsstellung fluchtet die Kopplungs-Anschlussformation 18 bevorzugt immer noch mit der Wechsel-Ankopplungsformation 30d, sodass die Kopplungs-Anschlussformation 18 durch Verlagerung derselben längs der Kopplungsbahn K auf die Wechsel-Ankopplungsformation 30d zu in einfacher Weise translatorisch in die Kopplungsstellung überführt werden kann.

Die Kopplungs-Anschlussformation 18 kann - wie die übrigen Fluidkanalträger-Anschlussformationen auch - durch eine Vorspanneinrichtung, etwa eine Schraubenfeder 64, längs der Kopplungsbahn K in die Kopplungsstellung vorgespannt sein. In diesem Falle weist der Fluidkanalträger 16 ein in den Figuren 1 bis 3 nicht dargestelltes Kraftgerät auf, welche alle Fluidkanalträger-Anschlussformationen gegen die Wirkung der ihnen jeweils zugeordneten Druckfeder in die ihnen ebenfalls jeweils zugeordneten Aufnahmeräume zurückzieht. Bevorzugt können die Fluidkanalträger-Anschlussformationen oder die sie tragenden Anschlussstutzen in der in den Fluidkanalträger 16 zurückgezogenen Stellung verriegelt werden, beispielsweise durch eine Bajonettformation.

Alternativ können die Fluidkanalträger-Anschlussformationen auch in die jeweilige in den Aufnahmeraum 62 zurückgezogene Stellung vorgespannt sein, etwa wenn die Schraubenfeder 64 eine Zugfeder ist. In diesem Falle kann zumindest die jeweils zur Ankopplung mit der Wechsel-Ankopplungsformation 30d ausgewählte Kopplungs-Anschlussformation 18 durch ein in den Figuren 1 bis 3 nicht dargestelltes Kraftgerät gegen die Wirkung der Vorspannfeder 64 von der Kopplungsbereitschaftsstellung in die in Figur 2 gezeigte Kopplungsstellung verstellt und gegebenenfalls in der Kopplungsstellung gegen eine Zurückbewegung in die Kopplungsbereitschaftsstellung verriegelt werden.

Eine der Fluidkanalträger-Anschlussformationen (siehe Anschlussstutzen 22 und 28 in den Figuren 1 und 3) kann durch Rotation des Fluidkanalträgers 16 relativ zum Leitungsbauteil 14 als Kopplungs-Anschlussformation ausgewählt werden, indem die jeweilige Fluidkanalträger-Anschlussformation längs einer Kreisbahn A als einer Auswahlbahn um die Rotationsachse R bewegt wird, bis die ausgewählte Kopplungs-Anschlussformation mit der Wechsel-Ankopplungsformation 30d fluchtet und sich somit in einer Kopplungsbereitschaftsstellung befindet.

Zur Erleichterung der Bewegung der Fluidkanalträger-Anschlussformationen um die Rotationsachse R ist bevorzugt ein Bewegungsantrieb 66, besonders bevorzugt ein elektromotorischer Bewegungsantrieb 66, am Leitungsbauteil 14 vorgesehen, welcher Bewegung und Kraft übertragend mit dem Fluidkanalträger 16 gekoppelt ist, beispielsweise über ein Zahnradgetriebe 68. Hierzu kann ein Teil der Außenumfangsfläche 16a (Mantelfläche) des Fluidkanalträgers 16 als Zahnrad oder Zahnkranz ausgebildet sein, vorzugsweise einstückig durch Kunststoffspritzguss.

Im Leitungsbauteil 14 ist eine Fluidleitungsanordnung 70 vorgesehen, die die jeweiligen Ankopplungsformationen 30a bis 30d durchsetzenden Fluidleitungsabschnitte 70a bis 70d (aus Gründen besserer Übersichtlichkeit sind nur die Fluidleitungsabschnitte 70a und 70d mit Bezugszeichen versehen) miteinander fluidleitend verbindet. Die nicht eigens gekennzeichneten Fluidleitungsabschnitte 70b und 70c durchsetzen die mit gleichen Kleinbuchstaben gekennzeichneten Ankopplungsformationen 30b bzw. 30c.

Die Fluidleitungsanordnung 70 verbindet die Fluidleitungsabschnitte 70a, 70b, 70c und 70d im dargestellten Beispiel der Figur 2 parallel zueinander in einer sogenannten Sternform. In dieser Ausgestaltung können jeweils zwei der Fluidleitungsabschnitte 70a bis 70d miteinander fluidleitend verbunden sein, ohne dass die Fluidleitung an den übrigen Fluidleitungsabschnitten entlang- oder an den Ankopplungsformationen vorbeiströmen muss.

Die dauerhaft mit den Ankopplungsformationen 30b und 30c gekoppelten Anschlussformationen 40b und 40c tragen in Figur 2 vorzugsweise kugelförmige Ventilkörper 72 (nur der Ventilkörper 72 an der Anschlussformation 40d ist mit einem Bezugszeichen versehen), welche bevorzugt ferromagnetisches Material umfassen oder aus ferromagnetischem Material gebildet sind. Das Leitungsbauteil 14 kann in an sich bekannter Weise verlagerbare Schaltmagnete aufweisen, welche in Zylinderführungen 74a bis 74d zur Annäherung an und Entfernung von den Ventilkörpern 72 geführt sein können, um die Ventilkörper 72 innerhalb der Fluidleitungsanordnung 70 durch Veränderung des lokal auf sie einwirkenden Magnetfelds zu verlagern. In Figur 3 ist ein ferromagnetischer Ventilkörper 72 an der Anschlussformation 36 des Zellkulturbehälters 12 gezeigt. Die in Figur 2 gezeigten zwei Ventilkörper bei vier Ankopplungsformationen 30a bis 30d sind ausreichend, um stets zwei Ankopplungsformationen und damit die sie durchsetzenden Fluidleitungsabschnitte in fluidleitender Kommunikation zu halten, während die übrigen zwei Ankopplungsformationen für eine Fluidleitung gesperrt sind. In Abwesenheit eines Magnetfelds von Schaltmagneten sind die Ventilkörper 72 durch Magnete 73a bis 73d in den Ventilsitzen in die jeweilige Schließstellung vorgespannt.

Beispielsweise kann die Fluidleitung 58 mit einem Entsorgungsbehälter gekoppelt sein und kann die Fluidleitung 60 mit einem Vorratsbehälter für Reinigungsfluid gekoppelt sein.

Die Fluidkanalstutzen 22, 28 sowie die nicht weiter mit Bezugszeichen versehenen Fluidkanalstutzen der Figuren 1 und 3 können mit unterschiedlichen Vorratsbehältern für Medien gekoppelt sein, beispielsweise mit einem Vorratsbehälter für Nährmedium, mit einem Vorratsbehälter für Medium zum Ablösen adhärenter Zellen von Innenflächen des Zellkulturbehälters und dergleichen.

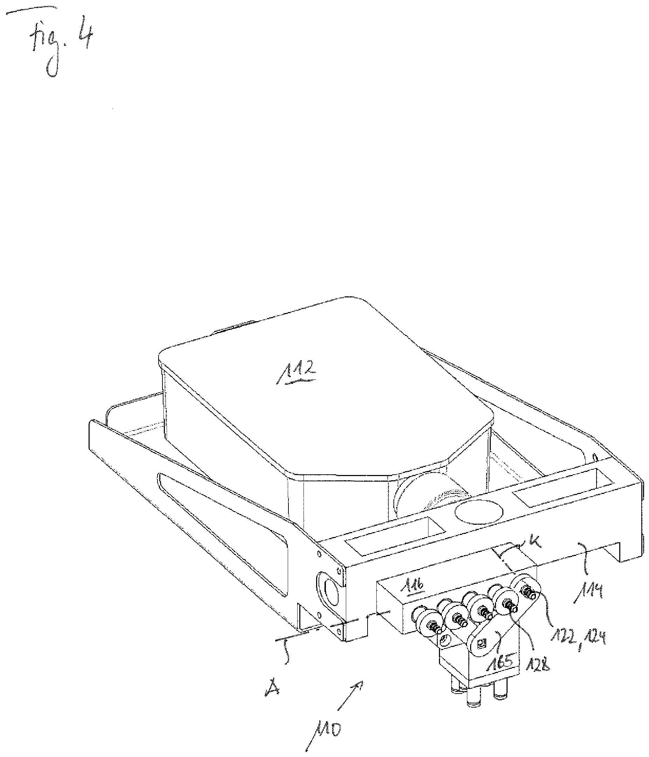

In den Figuren 4 bis 6 ist eine zweite Ausführungsform einer Fluid-Versorgungsschnittstelle der vorliegenden Erfindung dargestellt. Diese zweite Ausführungsform wird nachfolgend nur insoweit erläutert, als sie sich von der zuvor beschriebenen ersten Ausführungsform unterscheidet, auf deren Beschreibung ansonsten auch zur Erläuterung der zweiten Ausführungsform ausdrücklich verwiesen wird.

Gleiche und funktionsgleiche Bauteile und Bauteilabschnitte sind mit gleichen Bezugszeichen versehen wie in den Figuren 1 bis 3 der ersten Ausführungsform, jedoch erhöht um die Zahl 100.

Ein wesentlicher Unterschied zwischen der ersten und der zweiten Ausführungsform liegt darin, dass die zweite Ausführungsform einen relativ zum Leitungsbauteil 114 längs der Auswahlbahn A translatorisch verlagerbaren Fluidträger 116 aufweist. An diesem sind beispielhaft fünf Fluidkanalträger-Anschlussstutzen 122, 128 usw. vorgesehen, welche längs der Auswahlbahn A aufeinanderfolgend angeordnet sind. In dem dargestellten Beispiel ist der äußerste Anschlussstutzen 122 mit der Fluidkanalträger-Anschlussformation 118 als Kopplungs-Anschlussformation 118 ausgewählt. Ein Kraftgerät 165 (siehe Figur 4) sorgt für eine motorische Verstellbarkeit der Kopplungs-Anschlussformation 118 zwischen der in Figur 5 gezeigten Kopplungsstellung und einer Kopplungsbereitschaftsstellung, in welcher sich die übrigen vier Fluidkanalträger-Anschlussformationen 119 des Fluidkanalträgers 116 in Figur 5 befinden.

Da sich die Kopplungs-Anschlussformation stets relativ zum Leitungsbauteil 114 an derselben Stelle befindet, kann das Kraftgerät 165 relativ zum Leitungsbauteil 114 ortsfest vorgesehen sein. Es kann beispielsweise einen zwischen einer Eingriffsstellung und einer Außereingriffsstellung verschwenkbaren Schwenkarm aufweisen, welcher einenends einen Fluidkanalträger-Anschlussstutzen 122 der als Kopplungs-Anschlussformation 118 ausgewählten Fluidkanalträger-Anschlussformation 118 in Eingriff nehmen kann und nach der Ineingriffnahme längs der Kopplungsbahn K zusammen mit der in Eingriff genommenen Kopplungs-Anschlussformation verlagerbar ist.

Ein Bewegungsantrieb für die Verlagerung des Fluidkanalträgers 116 relativ zum Leitungsbauteil 114 längs der Auswahlbahn A ist in den Figuren 4 bis 6 nicht dargestellt.

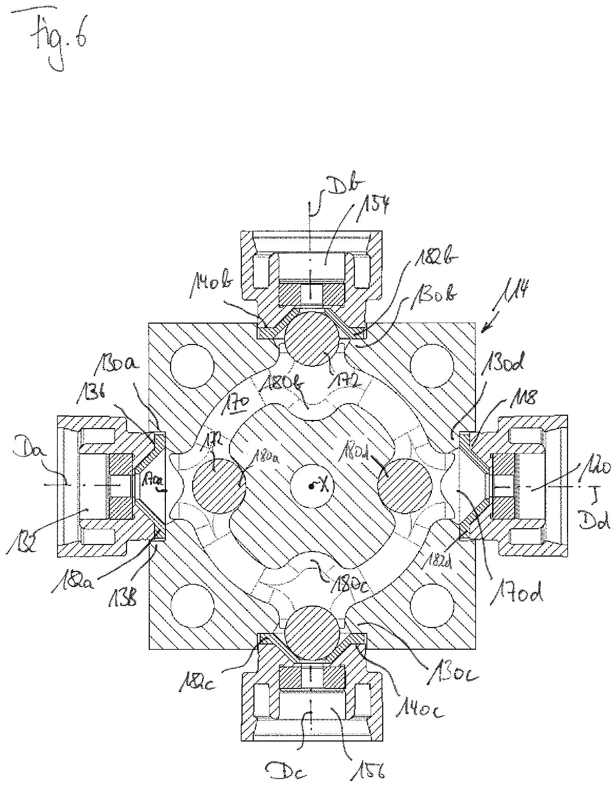

Ein weiterer Unterschied zwischen der ersten und der zweiten Ausführungsform liegt in der Ausgestaltung der Fluidleitungsanordnung 170. Diese ist in der zweiten Ausführungsform ringförmig ausgestaltet, d. h. die einzelnen die Ankopplungsformationen 130a bis 130d durchsetzenden Fluidleitungsabschnitte 170a bis 170d sind seriell miteinander gekoppelt. Das die Leitungsanordnung 170 tragende Teilbauteil des Leitungsbauteils 114 ist in Figur 6 vergrößert dargestellt.

Während in Figur 5 die einzelnen Leitungsabschnitte zwischen den einzelnen Ankopplungsformationen im Wesentlichen geradlinig ausgebildet sind, sind die gleichen Leitungsabschnitte in Figur 6 bogenförmig ausgebildet, gekrümmt um eine zentrale, zur Zeichenebene von Figur 6 orthogonale Krümmungsachse X.

Zur Aufnahme der Ventilkörper 172 nach deren Abheben vom jeweiligen Ventilsitz sind in der zweiten Ausführungsform Aufnahmeausnehmungen 180a bis 180d gebildet, welche zur geeigneten Aufnahme des konvex-sphärischen kugelförmigen Ventilkörpers 172 konkav-teilsphärisch ausgebildet sind. Die Aufnahmeausnehmungen 180a bis 180d sind orthogonal zur Mündungsöffnungsfläche der zugeordneten Ankopplungsformation 130a bis 130d (zugeordnete Ankopplungsformationen und Aufnahmeausnehmungen weisen den gleichen Kleinbuchstaben auf) in das die Fluidleitungsanordnung tragende Teilbauteil des Leitungsbauteils 114 hinein versetzt. Die Mündungsöffnungsflächen der einzelnen Ankopplungsformationen 130a bis 130b verlaufen im Wesentlichen eben und orthogonal zur Zeichenebene der Figur 4 und orthogonal zur jeweiligen Durchsetzungsrichtung Da bis Dd, längs welcher ein Fluidkanalabschnitt 132, 120, 154 und 156 die jeweiligen Anschlussformationen 118, 136, 140b und 140c durchsetzt.

Die Anschlussformationen 118, 119, 136, 140b und 140c weisen an ihren zum Leitungsbauteil 114 hinweisenden Längsenden Dichtungen 182a bis 182d auf, welche bevorzugt einstückig mit einem kegelförmig ausgestalteten Ventilsitz ausgebildet sind, auf dem die Ventilkugel 172 ruhen kann, wenn sie den jeweiligen Fluidkanalabschnitt der betreffenden Anschlussformation verschließt.

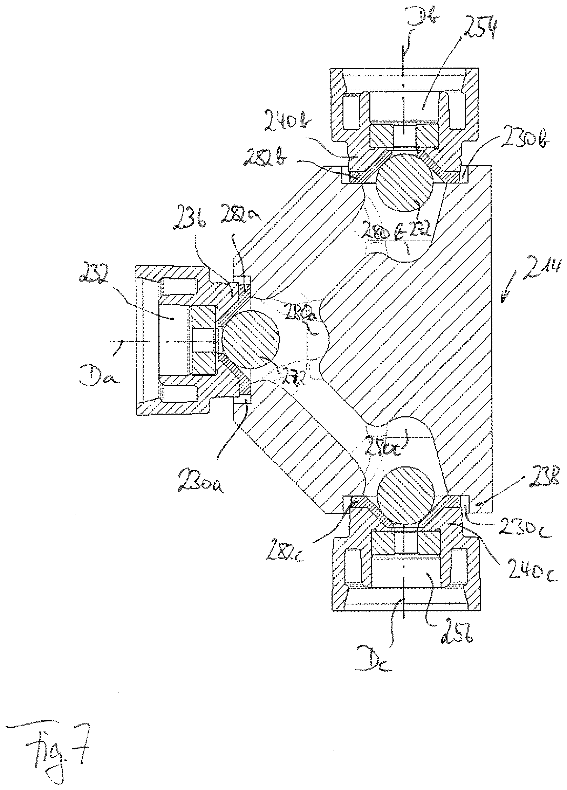

In Figur 7 ist eine weitere Ausführungsform eines eine Ventilleitungsanordnung 270 tragenden Teilbauteils des Leitungsbauteils 214 dargestellt.

Gleiche bzw. funktionsgleiche Bauteile bzw. Bauteilabschnitte wie in der zweiten Ausführungsform sind in der Ausführungsform von Figur 7 mit gleichen Bezugszeichen versehen, jedoch erhöht um die Zahl 100.

Wiederum gilt zur Erläuterung der Ausführungsform von Figur 7 die Beschreibung der vorhergehenden Ausführungsformen.

Die Ausführungsform von Figur 7 unterscheidet sich von jener der Figur 6 im Wesentlichen dadurch, dass die Verbindungskanäle, welche die einzelnen Fluidleitungsabschnitte 270a bis 270c miteinander verbinden, im Wesentlichen geradlinig ausgebildet sind. Die Fluidleitungsanordnung 270 ist in Figur 7 polygonal, aber nicht ringförmig ausgebildet.

Für die Ausführungsform sowohl der Figur 7 wie auch jener der Figur 6 gilt im Gegensatz zur ersten Ausführungsform, dass die Fluidleitungsabschnitte, welche die Fluidleitungsabschnitte 170a bis 170d bzw. 270a bis 207c verbinden, die die zugeordneten Ankopplungsformationen 130a bis 130d bzw. 230a bis 230c durchsetzen, in ihrer Querschnittsabmessung derart bemessen sind, dass der Ventilkörper 170 bzw. 270 nicht hindurchpasst und somit nicht von einer Ankopplungsformation zu einer anderen Ankopplungsformation gelangen kann.

In Figur 7 kann beispielsweise die Anschlussformation 240c als Kopplungs-Anschlussformation eines nicht dargestellten Fluidträgers ausgestaltet sein und kann die Ankopplungsformation 230c die Wechsel-Ankopplungsformation sein.

## Patentansprüche

1. Fluid-Versorgungsschnittstelle (10; 110; 210), insbesondere für eine Zellkulturanlage zur Einleitung eines Fluids in einen Zellkulturbehälter (12; 112) oder/und zur Ausleitung eines Fluids aus diesem, umfassend ein Leitungsbauteil (14; 114: 214) mit einer ersten Ankopplungsformation (30a; 130a; 230a) zur vorübergehenden Ankopplung eines ersten Fluidkanals (32; 132; 232), vorzugsweise eines Fluidkanals eines Zellkulturbehälters (12), mit einer zweiten Ankopplungsformation (30d; 130d; 230c) zur vorübergehenden Ankopplung eines zweiten Fluidkanals (20; 120; 256), vorzugsweise eines Fluidkanals eines Fluidvorrats, und mit einer dritten Ankopplungsformation (30b, 30c; 130b, 130c; 230b) zur vorübergehenden oder dauerhaften Ankopplung eines dritten Fluidkanals (54, 56; 154, 156; 254), vorzugsweise eines Entsorgungskanals, welche Ankopplungsformationen (30) jeweils von einem Fluidleitungsabschnitt (70a-70d; 170a-170d) durchsetzt sind, wobei in dem Leitungsbauteil (14; 114; 214) eine Fluidleitungsanordnung (70; 170; 270) ausgebildet ist, durch welche jeder Fluidleitungsabschnitt (70a-70d; 170a-170d) der ersten, der zweiten und der dritten Ankopplungsformation (30a-30d; 130a-130d; 230a-230c) mit jedem Fluidleitungsabschnitt (70a-70d; 170a-170d) der anderen beiden Ankopplungsformationen (30a-30d; 130a-130d; 230a-230c) zum Fluidtransport verbunden oder verbindbar ist,
**dadurch gekennzeichnet, dass** die Fluid-Versorgungsschnittstelle (10; 110; 210) weiter einen relativ zum Leitungsbauteil (14; 114; 214) beweglichen Fluidkanalträger (16; 116) aufweist, welcher wenigstens zwei gesondert voneinander ausgebildete und mit unterschiedlichen Fluidkanälen zum Fluidtransport verbundene, jeweils von einem Fluidkanalabschnitt (20; 120; 256) durchsetzte Fluidkanalträger-Anschlussformationen (18, 19; 118, 119) aufweist, und wobei eine der drei Ankopplungsformationen (30a-30d; 130a-130d;
230a-230c) als Wechsel-Ankopplungsformation (30d; 130d; 230c) zur vorübergehenden Herstellung einer gemeinsamen Fluidleitungsstrecke mit einer durch Relativbewegung von Leitungsbauteil (14; 114; 214) und Fluidkanalträger (16; 116) auswählbaren Fluidkanalträger-Anschlussformation (18, 19; 118, 119) ausgebildet ist.

2. Fluid-Versorgungsschnittstelle (10; 110; 210) nach Anspruch 1,
**dadurch gekennzeichnet, dass** der Fluidkanalträger (16; 116) wenigstens zwei Betriebsrelativstellungen aufweist, in denen jeweils eine andere Fluidkanalträger-Anschlussformation (18, 19; 118, 119) als Kopplungs-Anschlussformation (18; 118) ausgewählt und der Wechsel-Ankopplungsformation (30d; 130d; 230c) stärker angenähert ist als die jeweils andere Fluidkanalträger-Anschlussformation (19; 119).

3. Fluid-Versorgungsschnittstelle (10; 110; 210) nach Anspruch 2,
**dadurch gekennzeichnet, dass** sich die Kopplungs-Anschlussformation (18; 118) in dem Zustand stärkerer Annäherung an die Wechsel-Ankopplungsformation (30d; 130d; 230c) in einer Kopplungsbereitschaftsstellung befindet, in welcher der die Kopplungs-Anschlussformation (18, 19; 118, 119) durchsetzende Fluidkanalabschnitt (20; 120; 256) und der die Wechsel-Ankopplungsformation (30d; 130d; 230c) durchsetzende Fluidleitungsabschnitt (70d; 170d) miteinander fluchten oder dass sich die Kopplungs-Anschlussformation (18, 19; 118, 119) in dem Zustand stärkerer Annäherung an die Wechsel-Ankopplungsformation (30d; 130d; 230c) in einer Kopplungsstellung befindet, in welcher der die Kopplungs-Anschlussformation (18, 19; 118, 119) durchsetzende Fluidkanalabschnitt (20; 120) und der die Wechsel-Ankopplungsformation (30d; 130d; 230c) durchsetzende Fluidleitungsabschnitt (70d; 170d; 270c) zur Bildung einer gemeinsamen die Kopplungs-Anschlussformation (18, 19; 118, 119) und die Wechsel-Ankopplungsformation (30d; 130d; 230c) durchsetzenden Fluidleitungsstrecke gekoppelt sind.

4. Fluid-Versorgungsschnittstelle (10; 110; 210) nach Anspruch 3,
**dadurch gekennzeichnet, dass** die jeweilige Kopplungs-Anschlussformation (18, 19; 118, 119), vorzugsweise ohne Änderung der Relativstellung von Fluidkanalträger (16; 116) und Leitungsbauteil (14; 114; 214), zwischen der Kopplungsbereitschaftsstellung und der Kopplungsstellung verlagerbar ist.

5. Fluid-Versorgungsschnittstelle (10; 110; 210) nach Anspruch 4,
**dadurch gekennzeichnet, dass** die Kopplungs-Anschlussformation (18, 19; 118, 119) zwischen der Kopplungsbereitschaftsstellung und der Kopplungsstellung relativ zum Fluidkanalträger (16; 116) längs einer Kopplungsbahn (K) verlagerbar ist und gemeinsam mit dem Fluidkanalträger (16; 116) relativ zum Leitungsbauteil (14; 114; 214) längs einer durch ein Führungsmittel definierten Auswahlbahn (A) beweglich ist, wobei sich Kopplungsbahn (K) und Auswahlbahn (A) unterscheiden, vorzugsweise orthogonal zueinander sind.

6. Fluid-Versorgungsschnittstelle (10; 110; 210) nach Anspruch 4 oder 5,
**dadurch gekennzeichnet, dass** sie ein Kraftgerät (165) aufweist, durch welches die Kopplungs-Anschlussformation (18, 19; 118, 119) von der Kopplungsbereitschaftsstellung in die Kopplungsstellung verlagerbar ist, vorzugsweise gegen die Vorspannkraft einer Vorspanneinrichtung (64) verlagerbar ist.

7. Fluid-Versorgungsschnittstelle (10; 110; 210) nach einem der vorhergehenden Ansprüche,
**dadurch gekennzeichnet, dass** die Fluidleitungsanordnung (70; 170; 270) die Fluidleitungsabschnitte (70a-70d; 170a-170d) der ersten, der zweiten und der dritten Ankopplungsformation (30a-30d; 130a-130d; 230a-230c) des Leitungsbauteils (14; 114) parallel oder seriell miteinander verbindet, wobei die parallele Verbindung bevorzugt sternförmig und die serielle Verbindung bevorzugt bogenförmig oder polygonal, besonders bevorzugt ringförmig im Leitungsbauteil (14; 114; 214) ausgebildet ist.

8. Fluid-Versorgungsschnittstelle (10; 110; 210) nach einem der vorhergehenden Ansprüche,
**dadurch gekennzeichnet, dass** die Fluidleitungsanordnung (70; 170; 270) im Bereich wenigstens einer Ankopplungsformation (30a-30d; 130a-130d; 230a-230c), vorzugsweise in einer Richtung orthogonal zur Öffnungsfläche einer Mündung des die Ankopplungsformation (30a-30d; 130a-130d; 230a-230c) durchsetzenden Fluidleitungsabschnitts (70a-70d; 170a-170d) in der wenigstens einen Ankopplungsformation (30a-30d; 130a-130d; 230a-230c) in das Leitungsbauteil (14; 114; 214) hinein versetzt, eine Aufnahmeausnehmung (180a-180d; 280a-280c) aufweist, welche zur vorübergehenden Aufnahme eines Ventilkörpers (72; 172; 272) ausgebildet ist, insbesondere wenigstens abschnittsweise komplementär zu einem Oberflächenabschnitt des Ventilkörpers (72; 172; 272) ausgebildet ist, besonders bevorzugt konkav-teilsphärisch ausgebildet ist.

9. Fluid-Versorgungsschnittstelle (10; 110; 210) nach einem der vorhergehenden Ansprüche,
**dadurch gekennzeichnet, dass** die Mündung des Fluidleitungsabschnitts (70d; 170d) an der Wechsel-Ankopplungsformation (30d; 130d; 230c), bevorzugt auch an wenigstens einer weiteren Ankopplungsformation (30a-30c; 130a-130c; 230a-230b), von einer Dichtung (182a-182d; 282a-282c) oder von einer Anlagefläche, vorzugsweise von einer ebenen Anlagefläche, welche zur Anlage einer an einer Anschlussformation vorgesehenen Dichtung (182a-182d; 282a-282c) an ihr ausgebildet ist, umgeben ist.

10. Fluid-Versorgungsschnittstelle (10; 110; 210) nach einem der vorhergehenden Ansprüche,
**dadurch gekennzeichnet, dass** wenigstens eine der Ankopplungsformationen (30b-30c; 130b-130c; 230b) dauerhaft mit einer Anschlussformation (40b, 40c; 140b, 140c) zur Bildung einer gemeinsamen die Anschlussformation (40b, 40c; 140b, 140c) und die Ankopplungsformation (30b-30c; 130b-130c; 230b) durchsetzenden Fluidleitungsstrecke gekoppelt ist.

11. Fluid-Versorgungsschnittstelle (10; 110; 210) nach einem der vorhergehenden Ansprüche, **dadurch gekennzeichnet, dass** eine mit einer Ankopplungsformation (30a-30c; 130a-130c; 230a-230b) zur Bildung einer gemeinsamen Fluidleitungsstrecke koppelbare oder gekoppelte Anschlussformation (18, 19, 40b, 40c; 118, 119, 140b, 140c) einen Ventilsitz und einen Ventilkörper (72; 172; 272) aufweist, welcher in einem Betriebszustand mit für eine Fluiddurchleitung gesperrtem Fluidkanalabschnitt (20, 32, 54, 56; 120, 132, 154, 156; 232, 254, 256) der Anschlussformation (18, 19, 40b, 40c; 118, 119, 140b, 140c) auf dem Ventilsitz ruht.

12. Fluid-Versorgungsschnittstelle (10; 110; 210) nach Anspruch 11,
**dadurch gekennzeichnet, dass** der Ventilkörper (72; 172; 272) wenigstens teilweise, vorzugsweise vollständig, aus ferromagnetischem Material gebildet ist und dass der Ventilsitz einen Magneten (73a-73d; 173a-173d; 273a-273c), vorzugsweise ringförmigen Magneten (73a-73d; 173a-173d; 273a-273c), höchst bevorzugt einen den zugeordneten Fluidkanalabschnitt (20, 32, 54, 56; 120, 132, 154, 156; 232, 254, 256) umgebenden, von Fluid durchströmbaren Magneten (73a-73d; 173a-173d; 273a-273c) aufweist, welcher den Ventilkörper 72; 172; 272) magnetisch in eine Schließstellung vorspannt, in der der Ventilkörper (72; 172; 272) auf dem Ventilsitz ruht.

13. Fluid-Versorgungsschnittstelle (10; 110; 210) nach Anspruch 11 oder 12,
**dadurch gekennzeichnet, dass** wenigstens ein von der Ankopplungsformation (30a-30c; 130a-130c; 230a-230b) ausgehender und in Richtung zu einer weiteren Ankopplungsformation (30a-30c; 130a-130c; 230a-230b) hin verlaufender Verlagerungsabschnitt der Fluidleitungsanordnung (70; 170; 270) derart bemessen ist, dass er eine Verlagerung des Ventilkörpers (72; 172; 272) von der Ankopplungsformation (30a-30c; 130a-130c; 230a-230b) weg, vorzugsweise bis zu einer weiteren Ankopplungsformation (30a-30c; 130a-130c; 230a-230b), und zu dieser zurück gestattet.

14. Fluid-Versorgungsschnittstelle (10; 110; 210) nach einem der Ansprüche 11 bis 13,
**dadurch gekennzeichnet, dass** der Ventilkörper (72; 172; 272) ferromagnetisches Material aufweist, insbesondere aus ferromagnetischem Material hergestellt ist, und dass die Fluid-Versorgungsschnittstelle (10; 110) eine magnetische Schalteinrichtung (74a-74d) mit lokal an den Ankopplungsformationen (30a-30c; 130a-130c; 230a-230b) veränderbarer magnetischer Feldstärke aufweist, mit welcher der Ventilkörper (72; 172; 272) von dem Ventilsitz lösbar ist.

15. Fluid-Versorgungsschnittstelle (10; 110; 210) nach einem der vorhergehenden Ansprüche,
**dadurch gekennzeichnet, dass** der Fluidkanalträger (16; 116) relativ zum Leitungsbauteil (14; 114; 214) um eine Rotationsachse (R), vorzugsweise um eine zur Verlaufsrichtung des die Wechsel-Ankopplungsformation (30d; 130d; 230c) durchsetzenden Fluidleitungsabschnitts (70d; 170d) parallele Rotationsachse (R) drehbar ist, oder relativ zum Leitungsbauteil (14; 114) längs einer Translationsachse, vorzugsweise längs einer zur Verlaufsrichtung des die Wechsel-Ankopplungsformation (30d; 130d; 230c) durchsetzenden Fluidleitungsabschnitts (70d; 170d) orthogonale Translationsachse verschiebbar ist.

16. Zellkulturanlage mit wenigstens einem Zellkulturbehälter (12; 112), mit wenigstens zwei gesondert vorgesehenen Fluidvorräten, mit einer Fluidentsorgungssenke und mit einer Fluid-Versorgungsschnittstelle (10; 110) nach einem der vorhergehenden Ansprüche, wobei der Zellkulturbehälter (12; 112) eine Anschlussformation (36; 136; 236) aufweist, wobei die Ankopplungsverbindungen des Fluidkanalträgers (16; 116) fluidleitend mit den Fluidvorräten verbunden sind, wobei die erste Ankopplungsformation (30a; 130a; 230a) des Leitungsbauteils (14; 114; 214) zur Herstellung einer vorübergehenden Fluidtransportverbindung mit der Zellkulturbehälter-Anschlussformation (36 136; 236) ausgebildet ist, die zweite Ankopplungsformation (30d; 130d; 230c) des Leitungsbauteils (14; 114; 214) als die Wechsel-Ankopplungsformation (30d; 130d; 230c) zur Herstellung einer vorübergehenden Fluidtransportverbindung mit einer der Fluidkanalträger-Anschlussformationen (18) ausgebildet ist und die dritte Ankopplungsformation (30c) des Leitungsbauteils (14) zur Herstellung einer vorübergehenden oder dauerhaften Fluidtransportverbindung mit der Fluidentsorgungssenke ausgebildet ist.

## Claims

1. A fluid supply interface (10; 110; 210), in particular for a cell culture system for introducing a fluid into a cell culture container (12; 112) and/or for draining a fluid therefrom, comprising a line component (14; 114:214) having a first coupling formation (30a; 130a; 230a) for the temporary coupling of a first fluid channel (32; 132; 232), preferably a fluid channel of a cell culture container (12), a second coupling formation (30d; 130d; 230c) for the temporary coupling of a second fluid channel (20; 120; 256), preferably a fluid channel of a fluid reservoir, and a third coupling formation (30b, 30c; 130b, 130c; 230b) for the temporary or permanent coupling of a third fluid channel (54, 56; 154, 156; 254), preferably of a waste disposal channel, each of said coupling formations (30) being penetrated by a fluid line section (70a-70d; 170a- 170d), wherein in the line component (14; 114; 214), a fluid line assembly (70; 170; 270) is formed, by means of which each fluid line section (70a-70d; 170a-170d) of the first, second and third coupling formations (30a-30d; 130a-130d; 230a-230c) is or can be connected to each fluid line section (70a-70d; 170a-170d) of the other two coupling formations (30a-30d; 130a-130d; 230a-230c) for the purpose of fluid transport,
**characterized in that** the fluid supply interface (10; 110; 210) further has a fluid channel base (16; 116) which is movable relative to the line component (14; 114; 214) and which has at least two fluid channel base connector formations (18, 19; 118, 119), which are embodied as separate from one another and are connected to different fluid channels for the purpose of fluid transport, each said connector formation being penetrated by a fluid channel section (20; 120; 256), and wherein one of the three coupling formations (30a-30d; 130a-130d; 230a-230c) is embodied as a switchable coupling formation (30d; 130d; 230c) for the temporary production of a common fluid line segment with a fluid channel base connector formation (18, 19; 118, 119) that can be selected by a relative movement between line component (14; 114; 214) and fluid channel base (16; 116).

2. The fluid supply interface (10; 110; 210) according to claim 1, **characterized in that** the fluid channel base (16; 116) has at least two relative operating positions, in each of which a different fluid channel base connector formation (18, 19; 118, 119) is selected as the coupling connector formation (18; 118) and the switchable coupling formation (30d; 130d; 230c) is in closer proximity than the respectively other fluid channel base connector formation (19; 119).

3. The fluid supply interface (10; 110; 210) according to claim 2, **characterized in that** when the coupling connector formation (18; 118) is in closer proximity to the switchable coupling formation (30d; 130d; 230c), it is in a coupling ready position, in which the fluid channel section (20; 120; 256) that penetrates the coupling connector formation (18, 19; 118, 119) and the fluid line section (70d; 170d) that penetrates the switchable coupling formation (30d; 130d; 230c) are aligned with one another, or **in that** when the coupling connector formation (18, 19; 118, 119) is in closer proximity to the switchable coupling formation (30d; 130d; 230c), it is in a coupling position, in which the fluid channel section (20; 120) that penetrates the coupling connector formation (18, 19; 118, 119) and the fluid line section (70d; 170d; 270c) that penetrates the switchable coupling formation (30d; 130d; 230c) are coupled to form a common fluid line segment that penetrates the coupling connector formation (18, 19; 118, 119) and the switchable coupling formation (30d; 130d; 230c).

4. The fluid supply interface (10; 110; 210) according to claim 3, **characterized in that** each coupling connector formation (18; 19; 118, 119) can be displaced between the coupling ready position and the coupling position, preferably without changing the relative position between the fluid channel base (16; 116) and the line component (14; 114, 214).

5. The fluid supply interface (10; 110; 210) according to claim 4,
**characterized in that** the coupling connector formation (18, 19; 118, 119) can be displaced relative to the fluid channel base (16; 116) between the coupling ready position and the coupling position along a coupling path (K), and can be moved together with the fluid channel base (16; 116) relative to the line component 23 (14; 114; 214) along a selection path (A) defined by a guide means, wherein coupling path (K) and selection path (A) are different and are preferably orthogonal to one another.

6. The fluid supply interface (10; 110; 210) according to claim 4 or 5,
**characterized in that** it has a power unit (165) by means of which the coupling connector formation (18, 19; 118, 119) can be displaced from the coupling ready position to the coupling position, preferably against the prestressing force of a prestressing device (64).

7. The fluid supply interface (10; 110; 210) according to any of the preceding claims,
**characterized in that** the fluid line assembly (70; 170; 270) connects the fluid line sections (70a- 70d; 170a-170d) of the first, second, and third coupling formations (30a-30d; 130a-130d; 230a- 230c) of the line component (14; 114) to one another in parallel or in series, wherein the parallel connection is preferably configured as radial and the series connection is preferably configured as arcuate or polygonal, particularly preferably as ring-shaped in the line component (14; 114; 214).

8. The fluid supply interface (10; 110; 210) according to any of the preceding claims,
**characterized in that** the fluid line assembly (70; 170; 270) has a receiving recess (180a-180d; 280a-280c) in the region of at least one coupling formation (30a-30d; 130a-130d; 230a-230c), preferably offset into the line component (14; 114; 214) in a direction orthogonally to the opening surface of a mouth of the fluid line section (70a-70d; 170a-170d) that penetrates the coupling formation (30a-30d; 130a-130d; 230a-230c) in the at least one coupling formation (30a-30d; 130a- 130d; 230a-230c), said receiving recess being configured for the temporary accommodation of a valve body (72; 172; 272), in particular being configured as at least partially complementary to a section of the surface of the valve body (72; 172; 272), and particularly preferably being configured as concave/partially spherical.

9. The fluid supply interface (10; 110; 210) according to any of the preceding claims,
**characterized in that** the mouth of the fluid line section (70d; 170d) at the switchable coupling 24 formation (30d; 130d; 230c) and preferably also at least at one additional coupling formation (30a- 30c; 130a-130c; 230a-230b) is encompassed by a seal (182a-182d; 282a-282c) or by a contact surface, preferably by a planar contact surface, which is configured for the placement thereon of a seal (182a-182d; 282a-282c) that is provided on a connector formation.

10. The fluid supply interface (10; 110; 210) according to any of the preceding claims,
**characterized in that** at least one of the coupling formations (30b-30c; 130b-130c; 230b) is permanently coupled to a connector formation (40b, 40c; 140b, 140c) to form a common fluid line segment that penetrates the connector formation (40b, 40c; 140b, 140c) and the coupling formation (30b-30c, 130b-130c; 230b).

11. The fluid supply interface (10; 110; 210) according to any of the preceding claims,
**characterized in that** a connector formation (18, 19, 40b, 40c; 118, 119, 140b, 140c) that is or can be coupled to a coupling formation (30a-30c; 130a-130c; 230a-230b) to form a common fluid line segment has a valve seat and a valve body (72; 172; 272), which rests on the valve seat in an operating mode in which the fluid channel section (20, 32, 54, 56; 120, 132, 154, 156; 232, 254, 256) of the connector formation (18, 19, 40b, 40c; 118, 119, 140b, 140c) is blocked for fluid passage.

12. The fluid supply interface (10; 110; 210) according to claim 11,
**characterized in that** the valve body (72; 172; 272) is made at least partially, preferably entirely of ferromagnetic material, and **in that** the valve seat has a magnet (73a-73d; 173a-173d; 273a-273c), preferably a ring-shaped magnet (73a-73d; 173a-173d; 273a-273c), most preferably a magnet (73a-73d; 173a-173d; 273a- 273c) that encompasses the associated fluid channel section (20, 32, 54, 56; 120, 132, 154, 156; 232, 254, 256) and through which fluid can flow, which magnet magnetically prestresses the valve 25 body (72; 172; 272) into a closed position, in which the valve body (72; 172; 272) rests on the valve seat.

13. The fluid supply interface (10; 110; 210) according to claim 11 or 12,
**characterized in that** at least one displacement section of the fluid line assembly (70; 170; 270) that proceeds from the coupling formation (30a-30c; 130a-130c; 230a-230b) and extends toward a further coupling formation (30a-30c; 130a-130c; 230a-230b) is dimensioned such that it permits a displacement of the valve body (72; 172; 272) away from the coupling formation (30a-30c; 130a-130c; 230a- 230b), preferably up to a further coupling formation (30a-30c; 130a-130c; 230a-230b), and back.

14. The fluid supply interface (10; 110; 210) according to any of claims 11 to 13,
**characterized in that** the valve body (72; 172; 272) contains ferromagnetic material, in particular is made of ferromagnetic material, and **in that** the fluid supply interface (10; 110) has a magnetic switching device (74a-74d) with magnetic field strengths that can be adjusted locally at the coupling formations (30a-30c; 130a-130c; 230a-230b), and with which the valve body (72; 172; 272) can be released from the valve seat.

15. The fluid supply interface (10; 110; 210) according to any of the preceding claims,
**characterized in that** the fluid channel base (16; 116) is rotatable relative to the line component (14; 114; 214) about a rotational axis (R), preferably about a rotational axis (R) which is parallel to the orientation of the fluid line section (70d; 170d) that penetrates the switchable coupling formation (30d; 130d; 230c), or is displaceable relative to the line component (14; 114) along a translational axis, preferably along a translational axis which is orthogonal to the orientation of the fluid line section (70d; 170d) that penetrates the switchable coupling formation (30d; 130d; 230c).

16. A cell culture system having at least one cell culture container (12; 112), at least two separate fluid reservoirs, a fluid disposal sink and a fluid supply interface (10; 110) according to any of the preceding claims, wherein the cell culture container (12; 112) has a connector formation 26 (36; 136; 236), wherein the coupling connections of the fluid channel base (16; 116) are fluidically connected to the fluid reservoirs, wherein the first coupling formation (30a; 130a; 230a) of the line component (14; 114; 214) is configured to produce a temporary fluid transport connection with the cell culture container connector formation (36; 136; 236), the second coupling formation (30d; 130d; 230c) of the line component (14; 114; 214) is configured as the switchable coupling formation (30d, 130d; 230c) for producing a temporary fluid transport connection with one of the fluid channel base connector formations (18) and the third coupling formation (30c) of the line component (14) is configured to produce a temporary or permanent fluid transport connection with the fluid disposal sink.

## Revendications

1. Interface d'alimentation en fluide (10 ; 110 ; 210), en particulier pour l'établissement d'une culture de cellules pour introduire un fluide dans un récipient de culture de cellules (12 ;112) ou/et pour évacuer un fluide de celui-ci, comprenant un composant de conduite (14 ; 114 ; 214) avec une première structure d'accouplement (30a ; 130a ; 230a) pour l'accouplement temporaire d'un premier canal de fluide (32 ; 132 ; 232), de préférence d'un canal de fluide d'un récipient de culture de cellule (12), avec une deuxième structure d'accouplement (30d ; 130d ; 230c) pour l'accouplement temporaire d'un deuxième canal de fluide (20 ; 120 ; 256), de préférence d'un canal de fluide d'un réserve de fluide, et avec une troisième structure d'accouplement (30b, 30c ;130b, 130c ; 230b) pour l'accouplement temporaire ou permanent d'un troisième canal de fluide (54, 56 ; 154, 156 ; 254), de préférence d'un canal d'évacuation, une section de conduite de fluide (70a-70d ; 170a-170d) passant respectivement à travers les structures d'accouplement (30), un arrangement de conduite de fluide (70 ; 170 ; 270) étant formé dans le composant de conduite (14 ; 114 ; 214) par lequel chaque section de conduite de fluide (70a-70d ; 170a-170d) de la première, de la deuxième et de la troisième structure d'accouplement (30a-30d ; 130a-130d ; 230a-230c) est connectée ou peut être connectée avec chaque section de conduite de fluide (70a-70d ; 170a-170d) des autres deux structures d'accouplement (30a-30d ; 130a-130d ; 230a-230d),
**caractérisé en ce que**
l'interface d'alimentation en fluide (10 ; 110 ; 210) comprend en outre un support de canal de fluide (16 ; 116) mobile par rapport au composant de conduite (14 ; 114 ; 214), qui comprend au moins deux structures de connexion de support de canal de fluide (18, 19 ; 118, 119) formées séparément et connectées avec des canaux de fluide différents pour le transport de fluide, par lesquelles passe respectivement une section de canal de fluide (20 ; 120 ; 256), et où une des trois structures d'accouplement (30a-30d ; 130a-130d ; 230a-230c) est adaptée sous forme de structure d'accouplement d'échange (30d ; 130d ; 230c) pour l'établissement temporaire d'une ligne de conduite de fluide commune avec une structure de connexion de support de canal de fluide (18, 19 ; 118, 119) sélectionnable par un mouvement relatif du composant de conduite (14 ; 114 ; 214) et du support de canal de fluide (16 ; 116).

2. Interface d'alimentation en fluide (10 ; 110 ; 210) selon la revendication 1, **caractérisé en ce que** le support de canal de fluide (16 ; 116) comprend au moins deux positions opérationnelles relatives où une autre structure de connexion de support de canal de fluide (18, 19 ; 118, 119) est sélectionnée respectivement en tant que structure de connexion d'accouplement (18 ; 118) et est rapprochée plus fortement à la structure d'accouplement d'échange (30d ; 130d ; 230c) que l'autre structure de connexion de support de canal de fluide (19 ; 119).

3. Interface d'alimentation en fluide (10 ; 110 ; 210) selon la revendication 2, **caractérisé en ce que** dans un état de rapprochement plus fort à la structure d'accouplement d'échange (30d ; 130d ; 230c) la structure de connexion d'accouplement (18 ; 118) est dans une position de disponibilité à l'accouplement où la section de canal de fluide (20 ; 120 ; 256) passant à travers la structure de connexion de support de canal de fluide (18, 19 ; 118, 119) et la section de conduite de fluide (70d ; 170d) passant à travers la structure d'accouplement d'échange (30d ; 130d ; 230c) sont alignées ou **en ce que** la structure de connexion de support de canal de fluide (18, 19 ; 118, 119) est dans une position d'accouplement dans son état de rapprochement plus fort à la structure d'accouplement d'échange (30d ; 130d ; 230c), où la section de canal de fluide (20 ; 120) passant à travers la structure de connexion de support de canal de fluide (18, 19 ; 118, 119) et la section de conduite de fluide (70d ; 170d ; 270c) passant à travers la structure d'accouplement d'échange (30d ; 130d ; 230c) sont accouplées pour former ensemble une ligne de conduite de fluide passant à travers la structure de connexion de support de canal de fluide (18, 19 ; 118, 119) et la structure d'accouplement d'échange (30d ; 130d ; 230c).

4. Interface d'alimentation en fluide (10 ; 110 ; 210) selon la revendication 3, **caractérisé en ce que** la structure de connexion d'accouplement (18, 19 ; 118, 119) respective peut être déplacée entre la position de disponibilité à l'accouplement et la position d'accouplement, de préférence sans modification de la position relative du support de canal de fluide (16 ; 116) et du composant de conduite (14 ; 114 ; 214).

5. Interface d'alimentation en fluide (10 ; 110 ; 210) selon la revendication 4, **caractérisé en ce que** la structure de connexion d'accouplement (18, 19 ; 118, 119) peut être déplacée entre la position de disponibilité à l'accouplement et la position d'accouplement par rapport au support de canal de fluide (16 ; 116) le long d'une voie d'accouplement (K) et peut être déplacée avec le support de canal de fluide (16 ; 116) par rapport au composant de conduite (14 ; 114 ; 214) le long d'une voie de sélection (A) définie par un moyen de guidage, où la voie d'accouplement (K) et la voie de sélection (A) sont différentes, de préférence orthogonales entre elles.

6. Interface d'alimentation en fluide (10 ; 110 ; 210) selon les revendications 4 ou 5,
**caractérisé en ce qu'**elle comprend un dispositif de force (165) par lequel la structure de connexion d'accouplement (18, 19 ; 118, 119) peut être déplacée de la position de disponibilité à l'accouplement à la position d'accouplement, de préférence contre la force de précontrainte d'un dispositif de précontrainte (64).

7. Interface d'alimentation en fluide (10 ; 110 ; 210) selon une des revendications précédentes,
**caractérisé en ce que** l'arrangement de conduite de fluide (70 ; 170 ; 270) lie les sections de conduite de fluide (70a-70d ; 170a-170d) des premières, deuxièmes et troisièmes structures d'accouplement (30a-30d ; 130a-130d ; 230a-230c) du composant de conduite (14 ; 114) de manière parallèle ou sérielle, la liaison parallèle étant de préférence formée dans le composant de conduite (14 ; 114 ; 214) en étoile et la liaison parallèle étant de préférence en forme d'arc ou en forme polygonal, de manière particulièrement préférée en forme d'anneau.

8. Interface d'alimentation en fluide (10 ; 110 ; 210) selon une des revendications précédentes,
**caractérisé en ce que** l'arrangement de conduite de fluide (70 ; 170 ; 270) comprend, dans la région d'au moins une structure d'accouplement (30a-30d ; 130a-130d ; 230a-230c), de préférence dans un sens orthogonal à la surface d'ouverture d'une embouchure de la section de conduite de fluide (70a-70d ; 170a-170d) dans ladite au moins une structure d'accouplement (30a-30d ; 130a-130d ; 230a-230c) passant à travers la structure d'accouplement (30a-30d ; 130a-130d ; 230a-230c), décalée dans le composant de conduite (14 ; 114 ; 214), une encoche de réception (180a-180d ; 280a-280c) formée pour la réception temporaire d'un corps de vanne (72 ; 172 ; 272), de préférence partiellement complémentaire à une section de surface du corps de vanne (72 ; 172 ; 272), de manière particulièrement préférée concave ou partiellement sphérique.

9. Interface d'alimentation en fluide (10 ; 110 ; 210) selon une des revendications précédentes,
**caractérisé en ce que** l'embouchure de la section de conduite de fluide (70d ; 170d) est entourée à la structure d'accouplement d'échange (30d ; 130d ; 230c), de préférence aussi à au moins une autre structure d'accouplement (30a-30c ; 130a-130c ; 230a-230b) par un joint d'étanchéité (182a-182d ; 282a-282c) ou par une surface de contact, de préférence par une surface de contact plane, formée pour un contact d'un joint d'étanchéité (182a-182d ; 282a-282c) prévu à une structure de connexion d'accouplement.

10. Interface d'alimentation en fluide (10 ; 110 ; 210) selon une des revendications précédentes,
**caractérisé en ce qu'**au moins une des structures d'accouplement (30b-30c ;
130b-130c ; 230b) est accouplé de manière permanente avec une structure de connexion d'accouplement (40b, 40c ; 140b, 140c) pour former une ligne de conduite de fluide commune, passant à travers la structure d'accouplement (40b, 40c ; 140b, 140c) et la structure d'accouplement (30b-30c ; 130b-130c ; 230b).

11. Interface d'alimentation en fluide (10 ; 110 ; 210) selon une des revendications précédentes,
**caractérisé en ce qu'**une structure de connexion d'accouplement (18, 19, 40b, 40c ; 118, 119, 140b, 140c) qui est accouplée ou peut être accouplée à une structure d'accouplement (30a-30c ; 130a-130c ; 230a-230b) pour former une ligne de conduite de fluide commune, comprend une siège de vanne et un corps de vanne (72 ; 172 ; 272) qui, dans un état opérationnel, avec une section de canal de fluide (20, 32, 54, 56 ; 120, 132, 154, 156 ; 232, 254, 256) de la structure de connexion d'accouplement (18, 19, 40b, 40c ; 118, 119, 140b, 140c) bloquée pour un passage de fluide reste sur le siège de vanne.

12. Interface d'alimentation en fluide (10 ; 110 ; 210) selon la revendication 11, **caractérisé en ce que** le corps de vanne (72 ; 172 ; 272) est au moins en partie, de préférence entièrement fait d'un matériau ferromagnétique et **en ce que** le siège de vanne comprend un aimant (73a-73d ; 173a-173d ; 273a-273c), de préférence un aimant annulaire (73a-73d ; 173a-173d ; 273a-273c), de manière particulièrement préférée un aimant (73a-73d ; 173a-173d ; 273a-273c) entourant la section de canal de fluide (20, 32, 54, 56 ; 120, 132, 154, 156 ; 232, 254, 256) susceptible d'être parcouru par le fluide, pour précontraindre le corps de vanne (72 ; 172 ; 272) de manière magnétique dans une position d'arrêt où le corps de vanne (72 ; 172 ; 272) reste sur le siège de vanne.

13. Interface d'alimentation en fluide (10 ; 110 ; 210) selon la revendication 11 ou 12,
**caractérisé en ce qu'**au moins une section de déplacement de la section de conduite de fluide (70; 170 ; 270) partant de la structure d'accouplement (30a-30c ; 130a-130c ; 230a-230b) et s'étendant vers une autre structure d'accouplement (30a-30c ; 130a-130c ; 230a-230b) est dimensionnée de sorte qu'elle permet un déplacement du corps de vanne (72 ; 172 ; 272) s'éloignant de la structure d'accouplement (30a-30c ; 130a-130c ; 230a-230b), de préférence jusqu'à une autre structure d'accouplement (30a-30c ; 130a-130c ; 230a-230b) et de retour à celle-ci.

14. Interface d'alimentation en fluide (10 ; 110 ; 210) selon une des revendications 11 à 13,
**caractérisé en ce que** le corps de vanne (72 ; 172 ; 272) comprend du matériau ferromagnétique et est particulièrement fait d'un matériau ferromagnétique et **en ce que** l'interface d'alimentation en fluide (10 ; 110) comprend un mécanisme de commutation magnétique (74a-74d) avec une intensité de champ localement variable aux structures d'accouplement (30a-30c ; 130a-130c ; 230a-230b) par lequel le corps de vanne (72 ; 172 ; 272) peut être détaché du siège de vanne.

15. Interface d'alimentation en fluide (10 ; 110 ; 210) selon une des revendications précédentes,
**caractérisé en ce que** le support de canal de fluide (16 ; 116) peut tourner par rapport au composant de conduite (14 ; 114 ; 214) autour d'un axe de rotation (R), de préférence autour d'un axe de rotation (R) parallèle au sens d'extension de la section de conduite de fluide (70d; 170d) passant à travers la structure d'accouplement d'échange (30d ; 130d ; 230c), ou est déplaçable par rapport au composant de conduite (14 ; 114) le long d'un axe de translation, de préférence le long d'un axe de translation orthogonal au sens d'extension de la section de conduite de fluide (70d; 170d) passant à travers la structure d'accouplement d'échange (30d ; 130d ; 230c).

16. Dispositif de culture cellulaire avec au moins un récipient de culture cellulaire (12 ; 112) avec au moins deux stocks de fluide séparés avec une dépression de décharge de fluide et une interface d'alimentation en fluide (10 ; 110) selon une des revendications précédentes, le récipient de culture cellulaire (12 ; 112) comprenant une structure de connexion (36 ; 136 ; 236), les connexions d'accouplement du support de canal de fluide (16 ; 116) étant liées de manière conductrice de fluide avec les stocks de fluide, la première structure d'accouplement d'échange (30a ; 130a ; 230a) du composant de conduite (14 ; 114 ; 214) étant adaptée pour former une connexion temporaire de transport de fluide avec la structure de connexion (36 ; 136 ; 236) du récipient de culture cellulaire, la deuxième structure d'accouplement (30d ; 130d ; 230d) du composant de conduite (14 ; 114 ; 214) étant adaptée en tant que structure d'accouplement d'échange (30d ; 130d ; 230c) pour réaliser une connexion temporaire de transport de fluide avec une des structures de connexion de support de canal de fluide (18) et la troisième structure d'accouplement (30c) du composant de conduite (14) étant adaptée pour réaliser une connexion temporaire ou permanente de transport de fluide avec la dépression de décharge de fluide.
